# EUROPÄISCHE PATENTANMELDUNG

(11) **EP 0 739 627 A1**
(43) Veröffentlichungstag der Anmeldung: **30.10.1996**
(21) Anmeldenummer: 94109526.7
(22) Anmeldetag: 21.06.1994
(51) Int. Cl.: A61K 31/00, A61K 31/70, A61K 31/19

(54) **Verwendung von Glycyrrhizinsäure und ihrer Metabolite (Glycyrrhetinsäure) als Wirkstoff zur Herstellung eines Arzneimittels zur Therapie von malignen Erkrankungen des Menschen (Krebs)**

(71) Anmelder: Albrecht, Uwe, Dipl.-Dok., D-30938 Burgwedel (DE); AKUNA GESELLSCHAFT FÜR KLASSISCHE CHINESISCHE MEDIZIN UND ALTERNATIVMEDIZIN mbH, D-69429 Waldbrunn 1 (DE); Schuhmaier, Adolf, D-69429 Waldbrunn (DE)
(72) Erfinder: Die Erfindernennung liegt noch nicht vor

(57) **Zusammenfassung**

Der Wirkstoff Glycyrrhizinsäure wird innovativ zur Therapie maligner Erkrankungen des Menschen eingesetzt. Der Wirkstoff kennzeichnet sich durch hervorragende Verträglichkeit und Wirksamkeit und stellt auf diesem Gebiet eine wichtige Neuerung der medizinischen Therapiemöglichkeiten mit Arzneimitteln dar. Die Darreichungsformen dieses Arzneimittels sollen vor allem zur Injektion, aber auch zur oralen Gabe bestimmt sein.

## Beschreibung

### Technisches Gebiet, auf das sich die Erfindung bezieht

Die Erfindung bezieht sich auf das Gebiet der Medizin, hier auf Glycirrhizinsäure und ihre Metabolite, die aus der Süßholzwurzel (Glycyrrhiza glabra L.) gewonnen wird. Dieser extrahierte Wirkstoff soll nun bei der Herstellung von Arzneimitteln in verschieden Darreichungsformen als Wirkstoff Einsatz finden in der Therapie verschiedener maligner Erkrankungen des Menschen.

### Einschlägiger stand der Technik

Glycirrhizinsäure wird aus den Wurzeln von Glcyrrhizia glabra gewonnen, wobei dieser Stoff und seine Derivate den isolierten Terpenverbindungen der Gruppe der pentozyklischen Triterpene zuzuordnen ist. Das reine getrocknete Pulver ist von weißer Farbe mit einem stark süßlichen Geschmack, einem Schmelzpunkt von ca. 220 Grad Celsius und als wäßrige Lösung stark schäumend. Die verschiedenen Inhaltsstoffe der Süßholzwurzel werden in unterschiedlichen Gebieten der Technik verwendet:
- Textilindustrie,
- Lederindustrie,
- Tabakindustrie,
- Metallurgie,
- Lebensmittelindustrie (z.B. Bier-, Likör-, Bonbonproduktion)
- chemische Industrie:
- Seifen- und Kosmetikproduktion,
- Zusatz zu Mundwassern, Zahnpasten,
- Arzneimittelherstellung
Der Bestandteil der Süßholzwurzel Glycirrhizinsäure wird derzeit in der Arzneimittelherstellung als Geschmackskorrigens, Hilfsstoff, oder Wirkstoff verwendet. Bekannt ist hier die Herstellung von Arzneimitteln zur Therapie von gastrointestinalen Erkrankungen und Urologika. Die Wirkungen der Glycirrhizinsäure in diesen Gebieten sind anhand der Literatur ausreichend belegt und zudem teilweise traditionell überliefert.

### Würdigung des Standes der Technik

In der Therapie der malignen Erkrankungen des Menschen werden derzeit unterschiedliche Wirkstoffe eingesetzt. Nahezu alle gegenwärtig eingesetzten Wirkstoffe zeigen eine hohe Rate von unerwünschten Arzneimittelwirkungen und führen dabei häufig nicht zu dem gewünschten Therapieerfolg. Auch die Kombination verschiedener derzeit verfügbarer Wirkstoffe mindert die Lebensqualität des Krebskranken erheblich.

### Darstellung der Erfindung

Zur Herstellung eines Arzneimittels zur Therapie der malignen Erkrankungen des Menschen wird nun erfindungsgemäß Glycirrhizinsäure (bzw. deren Metabolite) als Wirkstoff eingesetzt. Hierbei wird vor allem eine injizierbare Zubereitung, die Glycirrhizin als aktiven Wirkstoff enthält, eingesetzt. Auch entsprechend hergestellte feste, zur oralen Einnahme bestimmte Arzneiformen mit dem Wirkstoff Glycirrhizin, kommen zum Einsatz.

### Vorteilhafte Wirkung der Erfindung

Die erfindungsgemäßen Arzneiformen können in einem weiten Indikationsbereich eingesetzt werden. Für die Behandlung von malignen Erkrankungen stehen derzeit nur mit hohen unerwünschten Wirkungen behaftete Arzneimittel zur Verfügung. Die erfindungsgemäßen Zubereitungen mit Glycirrhizinsäure haben bei hohem therapeutischen Wert eine geringe Rate von unerwünschten Arzneimittelwirkungen. Darüberhinaus sind derzeit keine den hohen ethischen Ansprüchen bei der Behandlung maligner Erkrankungen gerecht werdende Wirkstoffe verfügbar. Gerade in diesem Bereich stellt der Einsatz des Wirkstoffes Glycirrhizin zur Herstellung eines Arzneimittels einen bedeutenden Fortschritt der therapeutischen Möglichkeiten der Medizin dar.

### Beschreibung eines Weges zur Ausführung der Erfindung

Bei der Zubereitung der Injektionslösung liegt zunächst Glycirrhizin als weißes, kristallines Pulver mit stark süßlichem Geschmack vor. Dieses ist in kaltem Wasser nahezu unlöslich, wohl aber frei löslich in erwärmten, verdünnten Ethanol, erwärmter Essigsäure oder Ammoniumlösung.
Generika-Name: Glycirrhizin
Chemischer Name: 20 β-carboxy-11-oxo-30-norolean-12-en-3 β-yl-2-O-β-D-glucopyranuronosyl-β-D - glucopyranosiduronic- Säure
Molekularformel: C 42 - H 62 - O 16
Molekulargewicht: 822.92
Schmelzpunkt: 214 - 228 Grad Celsius
Es wird eine farblose, transparente, wässrige Lösung als injizierbare Lösung hergestellt. Der pH-Wert liegt zwischen 6.0 und 7.4. Die "Osmotic Pressure Ratio" bezogen auf physiologische Kochsalzlösung beträgt ca. 2.

Die fertige Lösung wird unter strikter Beachtung der "Good Manufacturing Practice" analysiert. Es werden dann additionale Wirkstoffe (z.B. Essigsäure und L-Cystein-Hydrochlorid) und Hilfsstoffe zugesetzt. Danach erfolgt unter sterilen Bedingungen die Abfüllung in Ampullen. Die so fertiggestellte Arzneiform ist zur intravenösen Verabreichung bestimmt.

## Patentansprüche

1. Alle Formen maligner Erkrankungen, vor allem Leberkarzinom und verschiedene Formen von Hautkrebs.
